# EUROPÄISCHE PATENTANMELDUNG

(11) **EP 2 193 780 A2**
(43) Veröffentlichungstag der Anmeldung: **09.06.2010**
(21) Anmeldenummer: 09177383.8
(22) Anmeldetag: 27.11.2009
(51) Int. Cl.: A61K 8/42, A61Q 5/08

(54) **Beschleunigte Aufhellmittel**

(30) Priorität: 08.12.2008 DE 102008060671
(71) Anmelder: Henkel AG & Co. KGaA, 40589 Düsseldorf (DE)
(72) Erfinder: Schmahl, Melanie, 40721, Hilden (DE); Goutsis, Konstantin, 41812, Erkelenz (DE); Sünger, Georg, 40547, Düsseldorf (DE)

(57) **Zusammenfassung**

Gegenstand der vorliegenden Anmeldung ist die kosmetische, nicht-therapeutische Verwendung von Harnstoff bzw. eines Harnstoff-Derivats in Aufhellmitteln für keratinische Fasern, insbesondere menschlicher Haare, zur Verbesserung der Penetration von Wasserstoffperoxid in die keratinischen Fasern, zur Beschleunigung der Aufhellwirkung von Wasserstoffperoxid sowie zur Reduktion der Faserschädigung beim Aufhellvorgang. Ein weiterer Gegenstand der vorliegenden Erfindung sind Aufhellmittel für keratinische Fasern, enthaltend in einem kosmetischen Träger, Harnstoff und/oder eines seiner Derivate.

## Beschreibung

Gegenstand der vorliegenden Anmeldung ist die kosmetische, nicht-therapeutische Verwendung von Harnstoff bzw. eines Harnstoff-Derivats in Aufhellmitteln für keratinische Fasern, insbesondere menschlicher Haare, zur Verbesserung der Penetration von Wasserstoffperoxid in die keratinischen Fasern, zur Beschleunigung der Aufhellwirkung von Wasserstoffperoxid sowie zur Reduktion der Faserschädigung beim Aufhellvorgang. Ein weiterer Gegenstand der vorliegenden Erfindung sind Aufhellmittel für keratinische Fasern, enthaltend in einem kosmetischen Träger, Harnstoff und/oder eines seiner Derivate.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Insbesondere für Blondierungen von dunkleren Ausgangshaarfarben besteht weiterhin ein Bedarf an leistungsfähigen Methoden. Die Grundlagen der Blondierverfahren sind dem Fachmann bekannt und in einschlägigen Monographien, z. B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Um eine ausreichenden Blondierwirkung zu erzielen, sind derartige Mittel üblicherweise stark alkalisch eingestellt, der pH-Wert liegt dabei zwischen 9 und 10,5. Derart hohe pH-Werte sind erforderlich, um eine Öffnung der äußeren Schuppenschicht (Cuticula) zu gewährleisten und somit eine Penetration der aktiven Spezies, in der Regel Wasserstoffperoxid, ins Haar zu ermöglichen. Als Alkalisierungsmittel wird üblicherweise Ammoniak eingesetzt, der allerdings den bekannten Nachteil des unangenehmen Geruchs und eventueller Reizung aufweist. In der Vergangenheit wurden häufig auch alternative Alkalisierungsmittel eingesetzt, die zwar in Abmischung mit Ammoniak selber akzeptable Blondierleistungen ergaben, diesen aber nicht vollständig ersetzen konnten. Es blieb daher bisher der Wunsch bestehen, die Ammoniakkonzentration zu reduzieren, ohne Abstriche bei der Blondierleistung der Mittel in Kauf nehmen zu müssen.

Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Um beim Bleichvorgang große Farbunterschiede zu erzielen, also insbesondere dunkle oder schwarze Haare zu entfärben, werden neben den bereits erwähnten Wasserstoffperoxidzubereitungen zumeist zusätzliche Bleichaktivatoren wie beispielsweise Persalze als zusätzliche Peroxidquellen, und/oder Carbonate als zusätzliche Alkalisierungsmittel benötigt.

Mit der oxidativen Aufhellung geht außerdem eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Aufhellmittel, welche eine gute Aufhellleistung zeigen, ohne gleichzeitig die Haarfaser zu schädigen, sind bislang nicht bekannt.

Zum Blondieren menschlicher Haare werden üblicherweise feste oder pastenförmige Zubereitungen mit festen Oxidationsmitteln unmittelbar vor der Anwendung mit einer verdünnten Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 60 Minuten. Es ist daher offensichtlich, dass beim Anwender das Bedürfnis nach Aufhellmitteln besteht, die die gewünschte Aufhellung in einer deutlich kürzeren zeit erzielen.

Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.

Im Rahmen ihrer Untersuchungen hat die Anmelderin nun überraschend gefunden, dass sich der Zusatz von Harnstoff und/oder einem seiner Derivate in Aufhellmitteln positiv im Bezug auf Verkürzung der Anwendungsdauer, Verbesserung der Penetration in die keratinische Faser und Verringerung der Haarschädigung auswirkt.

Ein erster Gegenstand der vorliegenden Erfindung ist die kosmetische, nicht-therapeutische Verwendung von Harnstoff und/oder einem seiner Derivate in Mitteln zum Aufhellen keratinischer Fasern zur Beschleunigung der Aufhellwirkung von Wasserstoffperoxid.

Unter keratinhaltigen bzw. keratinischen Fasern werden erfindungsgemäß Pelze, Wolle, Federn und insbesondere menschliche Haare verstanden. Obwohl die erfindungsgemäße Verwendung in erster Linie zum Färben und/oder Aufhellen von keratinhaltigen Fasern geeignet sind, steht prinzipiell einer Verwendung auch auf anderen Gebieten nichts entgegen.

Die erfindungsgemäßen Verwendungen betreffen Mittel, welche die Wirkstoffe in einem kosmetischen Träger enthalten. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als zwingenden Bestandteil enthält das Mittel zur erfindungsgemäßen Verwendung mindestens Harnstoff und/oder eines seiner Derivate.

Unter Derivaten des Harnstoffs sind Mono-, Di-, Tri- oder Tetra-substituierte Harnstoffderivate gemäß Formel (I) zu verstehen, 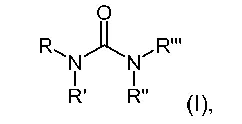 worin R bis R"' jeweils unabhängig voneinander für Wasserstoff, eine C₁-C₄-Alkylkette oder eine C₂-C₆-Hydroxyalkylkette stehen.

Bevorzugt stehen R bis R"' für Wasserstoff, Methyl und/oder 2-Hydroxyethyl. Besonders bevorzugt stehen R bis R"' jeweils für Wasserstoff, jeweils für Methyl, oder R steht 2-Hydroxyethyl und R' bis R'" stehen jeweils für Wasserstoff.

Ganz besonders bevorzugt enthält das Mittel Harnstoff (R bis R"' in Formel (I) stehen für Wasserstoff).

Es hat sich gezeigt, dass sich durch Mittel, enthaltend Harnstoff und/oder eines seiner Derivate die Anwendungsdauer von Aufhellmitteln auf keratinischen Fasern deutlich verkürzen lässt, ohne Einbußen in der Leistungsfähigkeit der Aufhellmittel hinnehmen zu müssen.

In maßgebliches Merkmal der vorliegenden Erfindung ist die Verwendung von Harnstoff und/oder einem seiner Derivate mit einer kurzen Anwendungsdauer eines Aufhellmittels auf menschlichem Haar. Neben einem Zeitgewinn für den Anwender lassen sich so gleichzeitig die Beeinträchtigungen für Kopfhaut und Haare durch das Aufhellmittel reduzieren.

Vorzugsweise beträgt die Anwendungsdauer so deutlich unter der Dauer eines handelsüblichen Aufhellmittels. Bevorzugt beträgt die Anwendungsdauer 3 bis 25 Minuten, insbesondere 5 bis 20 Minuten.

Ein weiterer Gegenstand der vorliegenden Erfindung ist daher die kosmetische, nicht-therapeutische Verwendung von Harnstoff und/oder einem seiner Derivate in Mitteln zum Aufhellen keratinischer Fasern zur Verbesserung der Aufhellwirkung von Wasserstoffperoxid in einer kurzen Anwendungsdauer von 3 bis 25 Minuten, bevorzugt von 5 bis 20 Minuten.

Ohne sich an diese Theorie zu binden, wird vermutet, dass der Zusatz von Harnstoff und/oder Harnstoff und/oder einem seiner Derivate einen positiven Einfluss auf die Penetration von Wasserstoffperoxid in die keratinische Faser besitzt.

Ein weiterer Gegenstand der vorliegenden Erfindung ist daher die kosmetische, nicht-therapeutische Verwendung von Harnstoff und/oder einem seiner Derivate in Mitteln zum Aufhellen keratinischer Fasern zur Penetrationsverbesserung von Wasserstoffperoxid in die keratinische Faser.

Wie bereits angedeutet wurde, geht mit der Verkürzung der Anwendungsdauer des Aufhellmittels auch die Belastung für die keratinische Faser zurück. Überraschend und in keiner Weise vorhersehbar wurde jedoch gefunden, dass der Zusatz von Harnstoff und/oder einem seiner Derivate an sich in Mittel zum Aufhellen von keratinischen Fasern bereits eine Reduktion der Faserschädigung erzielt.

Üblicherweise kann die durch Aufhellmittel bewirkte Schädigung der keratinischen Faser an dem Cysteinsäureanteil in der Faser ermittelt werden. Dabei werden Disulfid-Brücken innerhalb der Faser oxidativ gespalten und die resultierenden Thiol-Funktionalitäten des Cysteins oxidativ in Cysteinsäure-Einheiten umgewandelt. Der entstandene Cysteinsäureanteil in der keratinischen Faser ist mittels Nah-Infrarot-Spektroskopie (NIR) messbar und kann so zur Quantifizierung der Faserschädigung eingesetzt werden.

Ein weiterer Gegenstand der vorliegenden Erfindung ist daher die kosmetische, nicht-therapeutische Verwendung von Harnstoff und/oder einem seiner Derivate in Mitteln zum Aufhellen keratinischer Fasern zur Reduktion der Schädigung in der keratinischen Faser durch Wasserstoffperoxid.

Weitere Ausführungsformen der erfindungsgemäßen Verwendungen sind durch die nachfolgenden Erläuterungen und Merkmale der Mittel aufgezeigt.

Es hat sich gezeigt, dass in den Aufhellmitteln Harnstoff und/oder eines seiner Derivate in einer bestimmten Menge enthalten sein muss, um die genannten, positiven Effekte zu bewirken.

Ein weiterer Gegenstand der vorliegenden Erfindung ist daher ein Mittel zur Aufhellung keratinischer Fasern, enthaltend als Oxidationsmittel Wasserstoffperoxid, **dadurch gekennzeichnet, dass** es in einem kosmetischen Träger 0,01 bis 10 Gew.-%, bezogen auf das Gesamtgewicht des anwendungsbereiten Mittels, Harnstoff und/oder eines seiner Derivate enthält. Besonders bevorzugt enthält das Mittel Harnstoff und/oder eines seiner Derivate zwischen 0,5 und 5 Gew.-%, bezogen auf das Gesamtgewicht des anwendungsbereiten Mittels.

Üblicherweise enthalten Aufhellmittel für keratinische Fasern Oxidationsmittel zur oxidativen Zerstörung von natürlichen und/oder künstlichen Haarfarbstoffen. Ein bevorzugtes Oxidationsmittel ist dabei insbesondere Wasserstoffperoxid.

Es hat sich herausgestellt, dass der Zusatz von Ammoniumsalzen einer anorganischen oder organischen Säure den positiven Effekt der Mittel weiter verbessert. Solche Ammoniumsalze sind beispielsweise Ammoniumsulfat, Ammoniumchlorid, Ammoniumbromid, Ammmoniumcarbonat, Ammoniumcarbamat, Ammoniumformiat, Ammoniumhydrogencitrat, Ammoniumhydrogenoxalat, Ammoniumiodid, Ammoniumoxalat, Diammoniumhydrogenphosphat, Ammoniumdihydrogenphosphat, Ammoniumsulfamat oder Ammoniumtartrat.

Es ist bevorzugt, wenn dem erfindungsgemäßen Mittel das Ammoniumsalz in einem Anteil von 0,05 bis 5 Gew.-%, bezogen auf das Gesamtgewicht des erfindungsgemäßen Mittels, zugesetzt wird.

Ein besonders bevorzugtes Ammoniumsalz ist Ammoniumsulfat. Eine besonderen Ausführungsform des erfindungsgemäßen Mittels ist gegeben, wenn das Mittel zusätzlich 0,1 bis 3 Gew.-%, bevorzugt 0,3 bis 1,0 Gew.-%, jeweils bezogen auf das Gesamtgewicht des anwendungsbereiten Mittels, Ammoniumsulfat enthält.

Es hat sich gezeigt, dass es aus Gründen der Lagerstabilität zweckmäßig sein kann, das anwendungsbereite Aufhellmittel erst unmittelbar vor der Anwendung durch Vermischen von Komponenten herzustellen, um potentiellen Instabilitäten vorzubeugen.

Ein weiterer Gegenstand der vorliegenden Erfindung ist daher ein Mittel, welches dadurch gekennzeichnet ist, dass das Mittel unmittelbar vor der Anwendung durch Vermischen aus einer Oxidationsmittelzubereitung B, enthaltend in einem kosmetischen Träger mindestens ein Oxidationsmittel, und einer Emulsionszubereitung A sowie gegebenenfalls einer Blondaktivatorzubereitung C hergestellt wird, wobei mindestens eine der Zubereitungen A, B oder C 0,01 bis 10 Gew.-%, bezogen auf das Gesamtgewicht des anwendungsbereiten Mittels, Harnstoff und eines seiner Derivate enthält.

Eine bevorzugte Ausführungsform des erfindungsgemäßen Mittels ist **dadurch gekennzeichnet, dass** die Emulsionszubereitung A Harnstoff und eines seiner Derivate zu 0,01 bis 10 Gew.-%, bezogen auf das Gesamtgewicht des anwendungsbereiten Mittels, enthält.

Weiterhin kann es bevorzugt sein, wenn die Emulsionszubereitung A Ammoniumsulfat zu 0,3 bis 1,0 Gew.-%, bezogen auf das Gesamtgewicht des anwendungsbereiten Mittels, enthält.

Die erfindungsgemäßen Mittel zur Aufhellung keratinischer Fasern enthalten bevorzugt eine Oxidationsmittelzubereitung B. Diese enthält mindestens als Oxidationsmittel mindestens Wasserstoffperoxid.

Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n H₂O₂ (n ist eine positive ganze Zahl größer 0) und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 3 Gew.-%ige bis 12 Gew.-%ige Lösungen in Wasser verwendet.

Die erfindungsgemäßen Mittel enthalten mit besonderem Vorzug Wasserstoffperoxid. Hier sind erfindungsgemäße Mittel zur Blondierung keratinischer Fasern besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid, bezogen auf das Gesamtgewicht des erfindungsgemäßen Mittels enthalten.

Erfindungsgemäße Oxidationsmittelzubereitungen B sind vorzugsweise wässrige, fließfähige Oxidationsmittelzubereitungen. Dabei sind bevorzugte Zubereitungen **dadurch gekennzeichnet, dass** die fließfähige Oxidationsmittelzubereitung - bezogen auf ihr Gewicht - 40 bis 98 Gew.-%, vorzugsweise 60 bis 97,5 Gew.-%, besonders bevorzugt 70 bis 97 Gew.-%, weiter bevorzugt 75 bis 96 Gew.-% und insbesondere 80 bis 95 Gew.-% Wasser enthält.

Erfindungsgemäß kann aber die Oxidationsmittelzubereitung auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Geeignete Enzyme sind z. B. Peroxidasen, die die Wirkung geringer Mengen an Wasserstoffperoxid deutlich verstärken können.

Ein Einsatz bestimmter Metallionen oder -komplexe kann ebenfalls bevorzugt sein. Geeignete Metallionen sind beispielsweise Zn²⁺, Cu²⁺, Fe²⁺, Fe³⁺, Mn²⁺, Mn⁴⁺ , Li⁺, Mg²⁺, Ca²⁺, Ce⁴⁺, V³⁺, Co²⁺, Ru³⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate, Citrate und Tartrate. Durch Verwendung dieser Metallsalze kann die Blondierwirkung gezielt beeinflusst werden. Besonders bevorzugte Mittel enthalten diese Metallionen zu 0,0001 bis 2,5 Gew.-%, vorzugsweise 0,001 bis 1 Gew.-%, bezogen auf das Gesamtgewicht des erfindungsgemäßen Mittels.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab.

Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Oxidationsmittelzubereitung B enthält neben dem eigentlichen Oxidationsmittel und gegebenenfalls Komplexbildner weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methyl-propansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropan-sulfonsäure, das beispielsweise unter der Bezeichnung Rheothik^{®}11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester.

Die anionischen Acrylsäure- und/oder Methacrylsäure-Polymerisate oder -Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten.

Bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein bevorzugtes Handelsprodukt ist beispielsweise Aculyn 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn 22 sowie von der Firma National Starch unter den Handelsbezeichnungen Structure 2001 und Structure 3001 vertrieben.

Es kann erfindungsgemäß ebenfalls bevorzugt sein, wenn die anionischen Copolymere als ein Monomer Acrylsäureester mit längerkettigen Alkylresten enthalten. Solche Copolymere werden beispielsweise unter der INCI-Bezeichnung Acrylates/C10-30Allkylacrylate Crosspolymer vertrieben. Beispielhafte Handelsprodukte solcher Copolymere sind Carbopol ETD 2020, Carbopol Ultrez 20 und Pemulen TR-1 bzw. TR-2 der Firma Lubrizol.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäure-Gruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel 305 und Simulgel 600 der Firma SEPPIC enthalten. Die Verwendung dieser Verbindungen, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Weitere bevorzugte anionische Copolymere mit Sulfonsäuregruppen-haltigen Monomeren sind Copolymere mit dem Ammoniumsalz von 2-Acrylamido-2,2-dimethyl-ethylsulfonsäure. Bevorzugte Vertreter dieser Copolymere sind beispielsweise die Copolymere von Ammonium 2-Acrylamido-2,2-dimethyl-ethylsulfonat mit Vinylpyrrolidinon oder mit ethoxylierten Fettalkoholmethacrylsäureestern, wie Beheneth-25 methacrylat, die unter dem Handelsnamen Aristoflex AVC bzw. Aristoflex HMB von der Firma Clariant vertrieben werden.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnung Stabileze QM im Handel erhältlich.

Bevorzugt kann das erfindungsgemäße Mittel zusätzlich mindestens ein anionisches Acrylsäure-und/oder Methacrylsäure-Polymerisat oder -Copolymerisat. Bevorzugte Polymerisate dieser Art sind:
- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren,
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Gemäß einer zweiten Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloxy-ethyltrimethylammonium Chlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6) im Handel erhältlich.

Copolymere mit Monomereinheiten gemäß Formel (I) enthalten als nichtionogene Monomereinheiten bevorzugt Acrylamid, Methacrylamid, Acrylsäure-C₁-C₄-Alkylester und Methacrylsäure-C₁-C₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid-MethacroyloxyethyltrimethylammoniumChlorid-Copolymer. Solche Copolymere, bei denen die Monomeren in einem Gewichtsverhältnis von etwa 20:80 vorliegen, sind im Handel als ca. 50 %-ige nichtwässrige Polymerdispersion unter der Bezeichnung Salcare SC 92 erhältlich.

In einer dritten bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar C von der Firma Rhone Poulenc vertrieben. Erfindungsgemäß bevorzugte modifizierte Guargums enthalten C₁-C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar^{®} HP8, Jaguar HP60, Jaguar HP120, Jaguar DC 293 und Jaguar HP105 der Firma Rhone Poulenc im Handel erhältlich.

Weiterhin geeignete natürliche Verdickungsmittel sind ebenfalls bereits aus dem Stand der Technik bekannt.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize der Firma Amerchol und Natrosol der Firma Hercules vertrieben werden. Geeignete Carboxyalkylcellulosen sind insbesondere die Carboxymethylcellulosen, wie sie unter den Bezeichnungen Blanose von der Firma Aqualon, Aquasorb und Ambergum von der Firma Hercules und Cellgon von der Firma Montello vertrieben werden.

Bevorzugt sind weiterhin Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung lässt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁-C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze vertrieben wird.

Aber auch nichtionische, vollsynthetische Polymere, wie beispielsweise Polyvinylalkohol oder Polyvinylpyrrolidinon, sind als erfindungsgemäße Verdickungsmittel einsetzbar. Bevorzugte nicht-ionische, vollsynthetische Polymere werden beispielsweise von der Firma BASF unter dem Handelsnamen Luviskol vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel vertriebene Magnesiumschichtsilikat, sind bevorzugt.

Die gegebenenfalls zugesetzte Blondierzubereitung C des erfindungsgemäßen Mittels enthält einen zusätzlichen Bleichkraftverstärker.

Als zusätzliche Bleichkraftverstärker der Blondierzubereitung C können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die in der Blondierzubereitung C als Bleichkraftverstärker mindestens ein anorganisches Salz, ausgewählt aus Peroxomonosulfaten und/oder Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten. Der Einsatz von Persulfatsalzen bzw. Peroxodisulfatsalzen erfolgt in der Regel in Form eines gegebenenfalls entstaubten Pulvers, Paste oder eines in Form gepressten Formkörpers.

Die erfindungsgemäßen wasserfreien Zusammensetzungen anstelle und/oder zusätzlich zu den festen Peroxoverbindungen einen weiteren Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden.

Bleichverstärker vom Typ der Alkylcarbonate und -carbamate sowie Silylcarbonate und Silylcarbamate können in den wasserfreien Zusammensetzungen als Bleichverstärker eingesetzt werden und sind durch Verbindungen der Formel (BV) gekennzeichnet 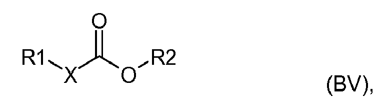 worin
- R1: für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht,
- X: für eine Gruppe O oder NR3 steht, worin R3 für ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silylgruppe oder für eine substituierte oder unsubstituierte Arylgruppe bzw. einen substituierten oder unsubstituierten Heterocyclus steht, und
- R2: für ein Wasserstoffatom, ein Alkalimetallatom, insbesondere Natrium, oder eine Gruppe SiR₃ in der die Reste R unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy- oder Aminogruppe stehen,

In Formel (BV) steht R1 vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Erfindungsgemäß besonders bevorzugt verwendete Zusammensetzungen sind **dadurch gekennzeichnet, dass** der Rest R1 in Formel (BV) ausgewählt ist aus Methyl, Ethyl, n-Propyl, i-Propyl, n-Butyl, i-Butyl, t-Butyl sowie Hydroxymethyl und Hydroxyethyl.

Bevorzugte Reste R2 und R3 in der Formel (BV) sind Wasserstoff, substituierte oder unsubstituierte, geradkettige oder verzweigte Alkylreste sowie Trialkylsilylreste. Unter diesen sind Wasserstoff, Methyl-, Ethyl-, t-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Obwohl prinzipiell keine Einschränkungen hinsichtlich der Formulierung der Blondierzubereitung C bestehen, hat es sich erfindungsgemäß als bevorzugt erwiesen, wenn die Zubereitung C wasserfrei formuliert ist. Wasserfrei im Sinne der vorliegenden Erfindung bedeutet einen Wassergehalt bezogen auf die Zubereitung C von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung C ist vorzugsweise als Pulver oder als wasserfreie Paste formuliert.

Im Falle der Formulierung als wasserfreie Paste hat es sich als besonders bevorzugt erwiesen, wenn die Zubereitung C mindestens einen nicht hydroxylierten Fettsäureester mit einem Schmelzpunkt von höchstens 50°C, insbesondere von höchstens 30°C, und/oder mindestens eine C₁₀-C₃₀-Fettsäure mit mindestens einer zusätzlichen Hydroxygruppe und/oder ein Derivat davon, enthält.

Ester von nicht hydroxylierten C₆-C₃₀-Alkylmonocarbonäuren mit C₂-C₃₀-Monoalkoholen eignen sich erfindungsgemäß bevorzugt als Fettsäureester. Bevorzugt sind die Monoester der Fettsäuren mit Monoalkoholen mit 2 bis 24 C-Atomen. Erfindungsgemäß besonders bevorzugt sind Isopropylmyristat (Rilanit IPM), Isononansäure-C16-18-alkylester (Cetiol SN), 2-Ethylhexylpalmitat (Cegesoft 24), Stearinsäure-2-ethylhexylester (Cetiol 868), Cetyloleat, Kokosfettalkohol-caprinat/- caprylat (Cetiol LC), n-Butylstearat, Oleylerucat (Cetiol J 600), Isopropylpalmitat (Rilanit IPP), Oleyloleat (Cetiol), Laurinsäurehexylester (Cetiol A), Myristylmyristat (Cetiol MM), Cetearylisononanoat (Cetiol SN), Ölsäuredecylester (Cetiol V).

Die gebrauchsfertigen Blondiermittel aus Emulsionszubereitung A und Oxidationsmittelzubereitung B sowie gegebenenfalls Blondierzubereitung C sollten bevorzugt einen pH-Wert im Bereich von 6 bis 12 aufweisen. Besonders bevorzugt ist die Anwendung im alkalischen Milieu. Die Anwendungstemperaturen können in einem Bereich zwischen 15 und 40 °C liegen. Nach einer Einwirkungszeit von 2 bis 60, bevorzugt 5 bis 45 Minuten wird das Blondiermittel durch Ausspülen von dem Haar entfernt. Das Nachwaschen mit einem Shampoo entfällt, wenn ein stark tensidhaltiger Träger verwendet wurde.

Erfindungsgemäß bevorzugte Blondiermittel für keratinische Fasern sind **dadurch gekennzeichnet, dass** sie einen alkalischen pH-Wert besitzen. Eine weitere bevorzugte Ausführungsform der vorliegenden Erfindung besteht darin, dass das anwendungsbereite Mittel einen pH-Wert zwischen 7,0 und 12,0, bevorzugt zwischen 8,0 und 11,0 besitzt.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Bei den pH-Werten im Sinne der vorliegenden Erfindung handelt es sich um pH-Werte, die bei einer Temperatur von 22 °C gemessen wurden.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrund-körper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Ein insbesondere bevorzugtes Alkanolamin ist Monoethanolamin.

Es hat sich aber im Rahmen der Untersuchungen zur vorliegenden Erfindung herausgestellt, dass erfindungsgemäß bevorzugte dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Die erfindungsgemäßen Zusammensetzungen enthalten die Alkalisierungsmittel bevorzugt in Mengen von 0,2 bis 25 Gew.-%, insbesondere 0,5 bis 10 Gew.-%.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wässriger Lösung vorliegen.

Erfindungsgemäß ganz besonders bevorzugt sind Wassergläser, die aus einem Silikat der Formel (SiO₂)ₙ(Na₂O)ₘ(K₂O)p gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt.

Es hat sich erfindungsgemäß als insbesondere vorteilhaft herausgestellt, wenn die Emulsionszubereitung A zusätzlich mindestens eine nichtionische Komponente mit einem HLB-Wert von kleiner oder gleich 8,0 enthält. Insbesondere aufgrund der vorteilhaften Stabilität der Zubereitungen wird die nichtionische Komponente mit einem HLB-Wert von kleiner oder gleich 8,0 jedoch bevorzugt in die Zubereitung A eingearbeitet.

Eine bevorzugte Ausführungsform der vorliegenden Erfindung ist daher ein Mittel, das dadurch gekennzeichnet ist, dass die Emulsionszubereitung A zusätzlich eine nichtionische Komponente mit einem HLB-Wert größer oder gleich 15,0 enthält.

Der HLB-Wert ist ein von Griffin (1950) eingeführtes Maß für die Wasser- bzw. Öllöslichkeit von Verbindungen, wobei Verbindungen mit niedrigen HLB-Werten hydrophobere Eigenschaften besitzen als Verbindungen mit hohen HLB-Werten. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Als nichtionische Komponenten mit einem HLB-Wert von größer oder gleich 15,0 lassen sich im Sinne der Erfindung nichtionische Emulgatoren einsetzen.

In einer bevorzugten Ausführungsform enthält das erfindungsgemäße Mittel einen nichtionischen Emulgator mit einem HLB-Wert von größer oder gleich 15,0. Unter nichtionischen Emulgatoren versteht man schließlich grenzflächenaktive Substanzen, die in wässriger Lösung keine Ionen bilden. Träger der grenzflächenaktiven Wirkung ist also das Gesamtmolekül. Die Hydrophilie solcher nichtionischer Emulgatoren wird durch den Anteil der polaren Gruppen im Molekül erreicht. Zu den nichtionischen Emulgatoren zählen Polymerisationsprodukte aus Ethylenoxid und Propylenoxid an gesättigte oder ungesättigte Fettalkohole, Fettsäureester mehrwertiger Alkohole mit gesättigten oder ungesättigten Fettsäuren, Alkylester von gesättigten oder ungesättigten Fettsäuren oder Alkylphenole und deren Alkoxylate.

Bevorzugte nichtionische Komponenten mit einem HLB-Wert von größer oder gleich 15,0 sind Ethylenglykolether von Fettalkoholen. Um einen HLB-Wert im erfindungsgemäßen Bereich zu erreichen, ist es bevorzugt, möglichst Ethylenglykolether mit Fettalkoholen mit hohem Ethoxylierungsgrad zu verwenden. Unter Ethoxylierungsgrad wird dabei die Molmenge an Ethylenoxid verstanden, die pro Mol Fettalkohol eingesetzt wurde. Bevorzugt ist dabei ein Ethoxylierungsgrad von wenigstens 15, vorzugsweise mindestens 20.

Erfindungsgemäß bevorzugte Ethylenglykolether sind beispielsweise Laureth-20, Laureth-25, Ceteth-20, Ceteth-25, Ceteth-30, Steareth-15, Steareth-20, Steareth-25, Steareth-30, Steareth-50, Ceteareth-15, Ceteareth-20, SCeteareth-25, Ceteareth-30 und Ceteareth-50. Insbesondere bevorzugt sind Ceteareth-20 und Ceteareth-30. Solche Verbindungen werden beispielsweise unter dem Handelsnamen Eumulgin B 2 (Ceteareth-20, HLB-Wert: 15,0) oder Eumulgin B 3 (Ceteareth-30, HLB-Wert: 17,0) von der Firma Cognis vertrieben.

Weiterhin bevorzugte nichtionische Komponenten mit einem HLB-Wert von größer oder gleich 15,0 sind Anlagerungsprodukte von Fettalkoholen mit Propylenoxid. Prinzipiell sind dabei Propylenglykolether der bereits genannten Fettalkohole einsetzbar, wobei Propylenglykolether mit Fettalkoholen mit hohem Propylenierungsgrad, vorzugsweise mindestens 15, bevorzugt sind. Unter Propylenierungsgrad wird dabei die Molmenge an Propylenoxid verstanden, die pro Mol Fettalkohol eingesetzt wurde.

Es ist weiterhin möglich, als nichtionische Komponenten mit einem HLB-Wert von größer oder gleich 15,0 gemischte Ethylen- und Propylenglykolether mit Fettalkoholen einzusetzen.

Als bevorzugte nichtionische Emulgatoren sind erfindungsgemäß ebenfalls Fettsäureester mehrwertiger Alkohole mit gesättigten oder ungesättigten Fettsäuren mit 8 bis 22, insbesondere 10 bis 18, Kohlenstoffatomen in der Fettsäuregruppe einsetzbar, sofern sie das erfindungsgemäße Kriterium eines HLB-Werts von größer oder gleich 15,0 erfüllen.

Als mehrwertige Alkohole sind dabei Ethylenglykol, Propylenglykol, Glycerin, Pentaerythrit, Sorbitan oder Zucker sowie Homo- oder Heterooligomere davon bevorzugt, insbesondere gemischte Ether von Zucker oder Sorbitan sowie Poylethylenglykol.

Bevorzugte, erfindungsgemäße nichtionische Komponenten sind Fettsäureester an Sorbitan als mehrwertigen Alkohol sowie Polyethylenglykol. Beispiele solcher Verbindungen sind Polyoxyethylen(20)sorbitanmonolaurat (HLB-Wert: 16,5), Polyoxyethylen(20)sorbitanmonopalmitat (HLB-Wert: 15,6) oder Polyoxyethylen(20)sorbitanmonooleat (HLB-Wert: 15,0).

Weiterhin ist es erfindungsgemäß bevorzugt, als nichtionische Komponenten Fettsäure- oder polyester mit Zuckern, insbesondere mit Saccharose, gegebenenfalls als ihre Anlagerungsprodukte mit Propylen- und/oder Ethylenoxid, einzusetzen, sofern diese Verbindungen einem HLB-Wert von größer oder gleich 15,0 besitzen.

Ester von nicht hydroxylierten C₆-C₃₀-Alkylmonocarbonäuren mit Polyethylenglykol lassen sich erfidnungsgemäß ebenfalls bevorzugt als nichtionische Verbindung mit einem HLB-Wert von Größer oder gleich 15 einsetzen. Beispiele dieser Verbindungen sind Polyoxyethylen(40)stearat (HLB-Wert: 16,3) oder Polyoxyethylen(40)monopalmitat (HLB-Wert: 15,6).

Eine weitere, erfindungsgemäß bevorzugte Klasse nichtionischer Komponenten mit einem HLB-Wert von größer oder gleich 15,0 stellen die Anlagerungsprodukte von Alkylphenolen an Ethylen-und/oder Propylenoxid dar. Bevorzugte Verbindungen tragen dabei 6 bis 21, insbesondere 6 bis 15, Kohlenstoffatome in der Alkylkette. Bevorzugte Vertreter für diese Verbindungen sind Octylphenol und Nonylphenol, alkoxyliert mit 10 bis 30 Mol Ethylen- und/oder Propylenoxid.

Es ist dabei bevorzugt, wenn die nichtionische Komponente mit einem HLB-Wert von größer oder gleich 15,0 zu 0,01 Gew.-% bis 10 Gew.-%, insbesondere zu 0,05 Gew.-% bis 8 Gew.-% und ganz besonders bevorzugt zu 0,1 Gew.-% bis 5 Gew.-%, jeweils bezogen auf das Gesamtgewicht des anwendungsbereiten Mittels, im erfindungsgemäßen Mittel vorliegt.

Besonders vorteilhafte, erfindungsgemäße Mittel enthalten zur zusätzlichen Verbesserung des Haarzustands einen Pflegestoff. Dieser zusätzliche Pflegestoff wird bevorzugt ausgewählt aus Vitaminen, Provitaminen und Vitaminvorstufen. Besonders bevorzugte Pflegestoffe sind dabei Vitamine, Provitamine und Vitaminvorstufen der Gruppen A, B₃, B₅, B₆, C, E, F und H. Aus Stabilitätsgründen wird der Pflegestoff vorzugsweise in die Emulsionszubereitung A eingearbeitet.

Ein weitere, bevorzugte Ausführungsform ist daher ein Mittel, das dadurch gekennzeichnet ist, dass die Emulsionszubereitung A zusätzlich einen Pflegestoff, ausgewählt aus Vitaminen, Provitaminen und Vitaminvorstufen, insbesondere solche der Gruppen A, B₃, B₅, B₆, C, E, F und H, enthält.

Es ist erfindungsgemäß bevorzugt, wenn der Pflegestoff, ausgewählt aus Vitaminen, Provitaminen und Vitaminvorstufen der Gruppen A, B₃, B₅, B₆, C, E, F und H, im Aufhellmittel in einem Anteil von 0,005 bis 2 Gew.-%, bevorzugt 0,01 bis 1,0 Gew.-%, jeweils bezogen auf das Gesamtgewicht des anwendungsbereiten Mittels, enthalten ist.

Die erfindungsgemäßen Mittel können zusätzlich mindestens eine farbverändernde Komponente, die bevorzugt ausgewählt wird
(1) aus mindestens einem Oxidationsfarbstoffvorprodukt
   und/oder
(2) aus mindestens einem direktziehenden Farbstoff.

Dabei kann es unerheblich sein, ob die farbgebende Komponente in die Emulsionszubereitung A, die Oxidationszubereitung B oder gegebenenfalls die Blondierzubereitung C eingearbeitet ist. Bevorzugt wird die farbgebende Komponente in die Emulsionszubereitung A eingearbeitet.

In Aufhellmitteln werden häufig farbverändernde Mittel eingesetzt, um einen sogenannten Mattierungseffekt zu erzielen. Unter Mattierung versteht der Fachmann die Fähigkeit eines Mittels, bei der Aufhellung in der Faser verbliebene Gelb- oder Roteindrücke durch komplementäre Haarfarben zu kompensieren. Häufig werden dazu geringere Mengen an farbgebenden Komponenten eingesetzt, vorzugsweise unter 1,0 Gew.-%, besonders bevorzugt weniger als 0,5 Gew.-% aller farbgebenden Komponenten, jeweils bezogen auf das Gesamtgewicht des erfindungsgemäßen Mittels.

Erfindungsgemäß bevorzugte Mittel zum Aufhellen keratinischer Fasern sind demnach **dadurch gekennzeichnet, dass** sie mindestens ein Oxidationsfarbstoffvorprodukt vom Entwicklertyp und/oder Kupplertyp enthalten.

Die erfindungsgemäßen Aufhellmittel enthalten als Oxidationsfarbstoffvorprodukte mindestens eine Kuppler- und mindestens eine Entwicklerkomponente. Zudem können die erfindungsgemäßen mattierenden Aufhellmittel auch noch direktziehende Farbstoffe als Nuanceure enthalten.

Bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen. Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,0001 bis 0,5 Gew.-%, vorzugsweise 0,001 bis 0,2 Gew.-%, jeweils bezogen auf das anwendungsbereite mattierende Aufhellmittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt: m-Aminophenol und/oder dessen Derivate, m-Diaminobenzol und/oder dessen Derivate, o-Diaminobenzol und/oder dessen Derivate, o-Aminophenolderivate, wie beispielsweise o-Aminophenol, Naphthalinderivate mit mindestens einer Hydroxygruppe, Di- beziehungsweise Trihydroxybenzol und/oder deren Derivate, Pyridinderivate, Pyrimidinderivate, Monohydroxyindol-Derivate und/oder Monoaminoindol-Derivate, Monohydroxyindolin-Derivate und/oder Monoaminoindolin-Derivate,Pyrazolonderivate (wie 1-Phenyl-3-methylpyrazol-5-on), Morpholinderivate (wie 6-Hydroxybenzomorpholin oder 6-Aminobenzomorpholin), Chinoxalinderivate (wie 6-Methyl-1,2,3,4-tetrahydrochinoxalin), sowie Gemische aus zwei oder mehreren Verbindungen aus einer oder mehreren dieser Klassen sind im Rahmen dieser Ausführungsform ebenso erfindungsgemäß.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diamino-phenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)-ethanol, 2-({3-[(2-Hydroxyethyl)amino]-4,5-dimethylphenyl}amino)ethanol, 2-[3-Morpholin-4-yl-phenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethyl-pyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen. Besonders bevorzugt ist dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das anwendungsbereite mattierende Aufhellmittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,0001 bis 0,2 Gew.-%, bevorzugt von 0,001 bis 0,1 Gew.-%, jeweils bezogen auf die gesamte Anwendungszubereitung, eingesetzt. Die Gesamtmenge an direktziehenden Farbstoffen beträgt vorzugsweise höchstens 0,1 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen sowie 3, 3',3",4,5,5',5",6-Octabromphenolsulfonphthalein (Tetrabromphenolblau).

Bevorzugte kationische direktziehende Farbstoffe sind dabei
kationische Triphenylmethanfarbstoffe, wie beispielsweise Basic Blue 7, Basic Blue 26, Basic Violet 2 und Basic Violet 14,
aromatischen Systeme, die mit einer quaternären Stickstoffgruppe substituiert sind, wie Basic Yellow 57, Basic Red 76, Basic Blue 99, Basic Brown 16 und Basic Brown 17, sowie
direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-di-nitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

In einer bevorzugten Ausführungsform der vorliegenden Erfindung enthält das erfindungsgemäße Mittel eine färbende Mattierungskombination, die eine Kombination aus mindestens einem blauen direktziehenden Farbstoff und einem roten direktziehenden Farbstoff umfasst, wobei das Gewichtsverhältnis zwischen der Summe aller blauen direktziehenden Farbstoffe und der Summe aller roten direktziehenden Farbstoffe einen Wert von größer oder gleich 1 besitzt.

Dadurch ist es möglich, unerwünschte Farbverschiebungen in Richtung rosa-/rose-farbene Nuancen zu vermieden.

Erfindungsgemäß bevorzugte Mittel sind **dadurch gekennzeichnet, dass** das Gesamtgewicht aller blauen direktziehenden Farbstoffe größer als das Gesamtgewicht aller roten direktziehenden Farbstoffe ist.

Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Es kann erfindungsgemäß weiterhin bevorzugt sein, wenn das Mittel weitere direktziehende Farbstoffe enthält. Besonders bevorzugt enthält das Mittel als weiteren direktziehenden Farbstoff einen gelben und/oder orangen Farbstoff.

Dies ist insbesondere dann vorteilhaft, wenn unerwünschte rötliche Farbverschiebungen beim Blondierprozess auftreten.

Eine bevorzugte Darreichungsform des erfindungsgemäßen Mittels des ist eine Verpackungseinheit (Kit-of-Parts), welche in getrennt voneinander konfektionierten Containern mindestens eine Oxidationsmittelszubereitung B, enthaltend mindestens ein Oxidationsmittel, ausgewählt aus Wasserstoffperoxid, und mindestens eine Emulsionszubereitung A enthält, wobei mindestens eine der Zubereitungen A und/oder B in einem kosmetischen Träger 0,01 bis 10 Gew.-%, bezogen auf das Gesamtgewicht des anwendungsbereiten Mittels, Harnstoff und eines seiner Derivate enthält.

Unter Container wird im Rahmen der vorliegenden Erfindung eine Umhüllung verstanden, die in Form einer gegebenenfalls wieder-verschließbaren Flasche, einer Dose, eines Tütchens, eines Sachets oder ähnlichen Umhüllungen vorliegt.

Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel.

Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Zubereitung A und der Oxidationsmittelzubereitung B gelten die obigen Ausführungen des vorgehenden Erfindungsgegenstands.

Für besonders starke Aufhellungen, insbesondere bei sehr dunklen, stark pigmentierten Ausgangshaarfarben, kann es jedoch erforderlich sein, zusätzliche Bleichkraftverstärker in das Mittel einzuarbeiten. Wird eine solche starke Aufhellung gewünscht, so ist es erfindungsgemäß bevorzugt, wenn zusätzlich eine Blondierzubereitung C, enthaltend mindestens einen Bleichkraftverstärker, der Mischung aus Oxidationsmittelzubereitung B und der Zubereitung A beigemischt wird.

Es kann dabei unerheblich sein, ob zunächst eine Mischung A und B hergestellt wird, und anschließend die Blondierzubereitung C zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.

Eine besonders bevorzugte Darreichungsform des Mittels ist weiterhin eine Verpackungseinheit (Kit-of-Parts), welche in getrennt voneinander konfektionierten Containern mindestens eine Oxidationsmittelzubereitung B, enthaltend Wasserstoffperoxid, mindestens einer Blondierzubereitung C, enthaltend mindestens einen Bleichkraftverstärker, und mindestens eine Zubereitung A enthält, wobei mindestens eine der Zubereitung A, B oder C in einem kosmetischen Träger 0,01 bis 10 Gew.-%, bezogen auf das Gesamtgewicht des anwendungsbereiten Mittels, Harnstoff und eines seiner Derivate enthält.

Hinsichtlich der bevorzugten Ausführungsführungsformen der Zubereitung A und der Oxidationsmittelzubereitung B und der Blondierzubereitung C gelten die obigen Ausführungen.

Die Mischung der Zubereitungen A und B bzw. gegebenenfalls der Zubereitungen A, B und C vor der Anwendung führt zu einer erfindungsgemäßen Anwendungsmischung.

Vorzugsweise sind die Oxidationsmittelzubereitung B und/oder die die Emulsionszubereitung A als fließfähigen Zubereitungen konfektioniert.

Vorzugsweise wird den fließfähigen Zubereitungen A und/oder B weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethyl-ammoniumglycinate, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylami-dopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Weiterhin hat es sich als vorteilhaft erwiesen, wenn die erfindungsgemäßen Haarbehandlungsmittel weitere, nichtionogene grenzflächenaktive Stoffe, die sie nicht unter die oben beschriebenen nichtionischen Komponenten mit einem HLB-Wert von 15,0 oder größer fallen, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe.

Als nichtionische Tenside eignen sich insbesondere C₈-C₂₂-Alkylmono- und oligoglycoside und deren ethoxylierte Analoga. Insbesondere die nichtethoxylierten Verbindungen haben sich als besonders geeignet erwiesen.Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid S 18 im Handel erhältliche Stearamidopropyl-dimethylamin dar.

Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Bevorzugt können die erfindungsgemäßen Zusammensetzungen mindestens einen nichtionogenen Emulgator bzw. Tensid mit einem HLB-Wert von 8 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionogene Emulgatoren bzw. Tenside mit einem HLB-Wert von 10-15 können erfindungsgemäß besonders bevorzugt sein.

Die erfindungsgemäßen Mittel können weiterhin alle für solche Zubereitungen bekannten Wirk-, Zusatz- und Hilfsstoffe enthalten. Weitere Wirk-, Hilfs- und Zusatzstoffe sind beispielsweise Strukturanten (wie Maleinsäure und Milchsäure), Proteinhydrolysate (insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate), Parfümöle, Cyclodextrine, Entschäumer (wie Silikone), Farbstoffe zum Anfärben des Mittels, Antischuppenwirkstoffe (wie Piroctone Olamine, Zink Omadine und Climbazol), Lichtschutzmittel (insbesondere derivatisierte Benzophenone, Zimtsäure-Derivate und Triazine), Wirkstoffe (wie Allantoin, Pyrrolidoncarbonsäuren und deren Salze sowie Bisabolol), Cholesterin, Konsistenzgeber (wie Zuckerester, Polyolester oder Polyolalkylether), weitere Fette und Wachse (wie Bienenwachs, Montanwachs und Paraffine), Fettsäurealkanolamide, Quell- und Penetrationsstoffe (wie Glycerin, Propylenglykolmonoethylether, Carbonate, Hydrogencarbonate, Guanidine sowie primäre, sekundäre und tertiäre Phosphate), Trübungsmittel (wie Latex, Styrol/PVP- und Styrol/Acrylamid-Copolymere), Perlglanzmittel wie (Ethylenglykolmono- und -distearat sowie PEG-3-distearat), Pigmente, Stabilisierungsmittel für Wasserstoffperoxid und andere Oxidationsmittel, Treibmittel (wie Propan-Butan-Gemische, N₂O, Dimethylether, CO₂ und Luft), Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der vorliegenden Erfindung ist ein Verfahren zur Blondierung menschlicher Haare, wobei ein Mittel durch Vermischen von mindestens einer Oxidationsmittelszubereitung B, enthaltend mindestens Wasserstoffperoxid, mindestens einer Blondierzubereitung C, enthaltend mindestens einen Bleichkraftverstärker, und mindestens einer Emulsionszubereitung A hergestellt wird, wobei mindestens eine der Zubereitungen A, B oder C in einem kosmetischen Träger 0,01 bis 10 Gew.-%, bezogen auf das Gesamtgewicht des anwendungsbereiten Mittels, Harnstoff und eines seiner Derivate enthält, das anwendungsbereite Mittel auf das Haar aufgetragen wird, für einen Zeitraum von 3 bis 25 Minuten, bevorzugt 5 bis 20 Minuten im Haar belassen wird, und das Haar anschließend mit Wasser und/oder einem handelsüblichen Shampoo gespült wird.

Im Rahmen dieses Gegenstandes der vorliegenden Erfindung gelten mutatis mutandis die oben getroffenen Aussagen analog.

So hat es sich beispielsweise als besonders vorteilhaft erwiesen, wenn die Zubereitungen A und B wässrig formuliert sind, wohingegen die Blondierzubereitung C vorzugsweise wasserfrei formuliert ist. Weiterhin ist es erfindungsgemäß bevorzugt, wenn die Zubereitung C pulverförmig ist.

Im Rahmen dieses Gegenstandes der vorliegenden Erfindung gelten mutatis mutandis die oben getroffenen Aussagen analog.

Die folgenden Beispiele sollen den Gegenstand der vorliegenden Erfindung näher erläutern ohne diesen in irgendeiner Weise zu beschränken.

### 1. Rezepturen

### 1.1 Emulsionszubereitungen A:

| **Rohstoffe** | **Gew.-%** | |
|---|---|---|
| | **A-E1** | **A-V1** |
| Hydrenol D | 11,00 | 11,00 |
| Lorol techn. | 2,75 | 2,75 |
| Eumulgin B2 | 0,25 | 0,25 |
| Texapon NSO | 26,50 | 26,50 |
| Ammoniumsulfat | 1,00 | 1,00 |
| Turpinal SL | 0,20 | 0,20 |
| Natriumsilikat 40 / 42 | 0,50 | 0,50 |
| Gluadin W40 | 0,35 | 0,35 |
| Vitamin F | 1,00 | 1,00 |
| Harnstoff, kristallin | 2,00 | --- |
| Ammoniak, 25% | 7,60 | 7,60 |
| Parfum | qs | qs |
| Wasser | ad 100 | ad 100 |

### 1.2 Oxidationszubereitungen B:

| **Rohstoff** | **Gew.-%** | |
|---|---|---|
| | **B-E1** | **B-V1** |
| Ammoniak 25 % | 0,62 | 0,62 |
| Dipicolinsäure | 0,10 | 0,10 |
| Dinatriumpyrophosphat | 0,03 | 0,03 |
| Turpinal SL | 1,50 | 1,50 |
| Texapon NSO | 2,00 | 2,00 |
| Dow Corning DB 110 A | 0,07 | 0,07 |
| Aculyn 33A | 12,00 | 12,00 |
| Harnstoff, kristallin | 2,00 | --- |
| Wasserstoffperoxid 50 % | 22,40 | 22,40 |
| Wasser | ad 100 | ad 100 |

### 1.3 Blondieraktivatorzubereitung C:

| **Rohstoff** | **C [Gew.-%]** |
|---|---|
| Harnstoff, kristallin | 98,0 |
| Kieselsäure, pyrogen | 2,0 |

- Hydrenol D: C₁₆-C₁₈-Fettalkohol (INCI-Bezeichnung: Cetearyl alcohol) (Cognis)
- Lorol tech.: C₁₂-C₁₈-Fettalkohol (INCI-Bezeichnung: Coconut alcohol) (Cognis)
- Eumulgin B2: C₁₆-C₁₈-Fettalkohol, ethoxyliert (20 EO) (INCI-Bezeichnug: Ceteareth20) (Cognis)
- Texapon NSO: Laurylethersulfat, Natriumsalz (ca. 27,5% Aktivsubstanz; INCI-Bezeichnung: Sodium Laureth Sulfate) (Cognis)
- Turpinal SL: 1-Hydroxyethan-1,1-diphosphonsäure (ca. 60% Aktivsubstanzgehalt; INCI-Bezeichnung: Etidronic Acid, Aqua (Water)) (Solutia)
- Natriumsilikat 40/42: Natronwasserglas
- Gluadin W 40: hydrolysiertes Weizenprotein (ca. 40% Aktivsubstanzgehalt; INCI-Bezeichnung: Hydrolyzed Wheat Gluten / Hydrolyzed Wheat Protein) (Cognis)
- Aculyn 33: Acrylpolymer (ca. 28% Festkörper in Wasser; INCI-Bezeichnung: Acrylates Copolymer) (Rohm & Haas)
- Dow Corning DB 110 A: nicht ionische Silikonemulsion (INCI-Bezeichnung: Dimethicon) (Dow Corning)

Die Emulsionszubereitung A wurde jeweils im Verhältnis 1:1 mit der Entwicklerdispersion B ausgemischt. Im Fall der Zugabe einer Blondieraktivatorzubereitung C betrug das Mischungsverhältnis im anwendungsbereiten Mittel 47,9 Gew.-% an Emulsionszubereitung A, 50,5 Gew.-% an Oxidationszubereitung B und 1,6 Gew.-% an Blondieraktivatorzubereitung C.

Der pH-Wert der fertigen Anwendungsmischung lag zwischen 9 und 10,2.

### 2. Aufhellergebnisse

Die anwendungsbereiten Mittel wurden für den Blondierprozess wurde auf Strähnen hellbraunen Haares (Firma Fischbach) von ca. 0,7 g Gewicht die 4-fache Menge der fertigen Anwendungsmischung appliziert, für die angegebene Einwirkzeit bei 32 °C auf dem Haar belassen, und anschließend mit einem handelsüblichen Shampoo gewaschen und mit einem Föhn getrocknet.

Die farbmetrischen Messungen erfolgten an jeweils 8 Messpunkten pro Strähne. Als Messgerät diente der Spectralflash SF 450 der Firma Datacolor. Folgende Messparameter wurden dabei verwendet:
- mit Glanz
- Messblende SAV
- D65 (Tageslicht)
- 10° Beobachter
Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung.

Es zeigte sich, dass es für das Aufhellergebnis unerheblich ist, ob das erfindungsgemäße, anwendungsbereite Mittel Harnstoff in der Emulsionszubereitung A, in der Oxidationszubereitung B oder durch Zugabe einer Blondierzubereitung C enthält.

Die nachfolgenden Messwerte (Tabelle 1) belegen den vorteilhaften Blondiereffekt des erfindungsgemäßen Mittels nach der kurzen Einwirkzeit von lediglich 10 Minuten.

| **Probe** | **Einwirkzeit [min]** | **L** | Δ**L(unbeh.-beh.)** |
|---|---|---|---|
| unbehandelt | | 25,85 | |
| A-V1 + B-V1 | 10 | 36,26 | 10,41 |
| A-E1 + B-V1 (1,0 Gew.-% Harnstoff) | 10 | 38,65 | 12,80 |
| A-V1 + B-V1 | 20 | 35,07 | 9,22 |
| A-E1 + B-V1 (1,0 Gew.-% Harnstoff) | 20 | 37,87 | 12,02 |

### 3. Auswertung der Haarschädigung

### 3.1 Methodenbeschreibung zur NIR-Analytik von behandeltem Haar

### Qualitative Bestimmung der Haarstrukturschädiqunq mittels Clusteranalyse

Haare können durch oxidative Behandlung in Aufhellmitteln geschädigt werden.

Cystin ist ein Bestandteil des menschlichen Haares. Cystin zeigt bei Spektrum bei einer Wellenlänge von 6200 cm⁻¹ - 5500 cm⁻¹ typische Schwingungen im NIR (Nah-Infra-Rot). Die in Cystin enthaltene Disulfid-Brücke wird bei einer Haarbehandlung oxidativ gebrochen, und der resultierende Thiol durch weitere Oxidation in die Sulfonsäure-Gruppe der Cysteinsäure überführt. Gleichzeitig verändert sich das Haar hinsichtlich des Cysteinsäuregehalts, welcher mit stärker werdender, oxidativer Schädigung zunimmt. Cysteinsäure zeigt sich im NIR-Spektrum bei einer Wellenlänge von 5020 cm⁻¹ - 4020 cm⁻¹. Die Abnahme an Cystin und die Zunahme an Cysteinsäure stellt daher einen Indikator für die Schädigung des Haares dar.

Die Aufnahme der Spektren wird mit einem MPATM FT-NIR-Spektrometer der Bruker Optik GmbH (Ettlingen, BRD) durchgeführt. Der Nah-Infrarot (NIR)-Bereich umfasst den Wellenzahlbereich von 12500 cm⁻¹ bis 4000 cm⁻¹ und ist für Oberton- und Kombinationsschwingungen von z.B. CH-, OH- und NH-Gruppen charakteristisch. Die Messung der Proben erfolgt mit dem Integrationskugelmodul an sechs verschiedenen Probenpositionen in diffuser Reflexion. Für die Analyse der gemessenen NIR-Spektren wird der Wellenzahlbereich 8000 cm⁻¹ - 4020 cm⁻¹ gewählt. Für die chemometrische Auswertung wird das geräteunabhängige Softwarepaket Unscrambler (Fa. CAMO, Oslo, Norwegen) verwendet.

Anhand einer PCA (Principal Compound Analysis) kann man die Separation der Spektren in sogenannte "Cluster" erkennen Je größer die Abstände der Cluster zu dem der unbehandelten Muster sind, desto größer ist die spektrale Abweichung einhergehend mit dem Ausmaß der Haarstrukturveränderung. Auf diese Art und Weise lassen sich die Haare nach Behandlung mit Aufhellern und anschließender Vermessung im NIR in Cluster einteilen. Die Stärke der Veränderung des behandelten Haares ist sofort erkennbar.

### Quantitative Bestimmung der Haarstrukturschädigung mittels Kalibrationserstellung

NIR-Spektren lassen sich auch quantitativ auswerten, wenn Referenzwerte vorhanden sind. Bei der Modellbildung wird mit Hilfe eines Partial Least Squares (PLS)-Algorithmus eine Korrelation zwischen spektroskopischen Daten und den entsprechenden Konzentrationswerten der einzelnen Komponenten berechnet. Referenzwerte erhält man, indem man Proben im Labor über ein anderes unabhängiges analytisches Verfahren quantitativ bestimmt. Das PLS-Modell wird durch Veränderung des Frequenzbereiches, mathematische Datenvorbehandlung, Auswahl der geeigneten Faktorenzahl und automatischer Ausreißererkennung verbessert.

Eine detaillierte Beschreibung der Methode findet sich in Evaluation of Physical Properties of Human Hair by Diffuse Reflectance Near-Infrared Spectroscopy, Y. Miyamae, Y. Yamakawa und Y. Ozaki in Applied Spectroscopy, 2007, Vol. 61 (2), S. 212 ff.

### 3.2 Ergebnisse

| Haarprobe | | Einwirkzeit [min] | NIR-Analsyenwert Cysteinsäure [mol/100 mol Aminosäure] |
|---|---|---|---|
| Kerling 6/0 | A-V1 + B-V1 | 10 | 1,1 |
| | A-E1 + B-V1 (1,0 Gew.-% Harnstoff) | 10 | 1,0 |
| | A-V1 + B-V1 | 20 | 1,1 |
| | A-E1 + B-V1 (1,0 Gew.-% Harnstoff) | 20 | 0,9 |

Die Cysteinsäuregehalte der Haarproben wurden mittels NIR-Messungen bestimmt. Die Ergebnisse zeigen für beide Einwirkzeiten deutlich geringere Mengen an Cysteinsäure für das erfindungsgemäße Aufhellmittel verglichen mit den Vergleichsaufhellmitteln. Dadurch wird die signifikant geringere Haarschädigung durch die erfindungsgemäße Aufhellzusammensetzung verdeutlicht.

## Patentansprüche

1. Kosmetische, nicht-therapeutische Verwendung von Harnstoff und/oder einem seiner Derivate in Mitteln zum Aufhellen keratinischer Fasern zur Beschleunigung der Aufhellwirkung von Wasserstoffperoxid.

2. Kosmetische, nicht-therapeutische Verwendung von Harnstoff und/oder einem seiner Derivate in Mitteln zum Aufhellen keratinischer Fasern zur Verbesserung der Aufhellwirkung von Wasserstoffperoxid in einer kurzen Anwendungsdauer von 3 bis 25 Minuten, bevorzugt von 5 bis 20 Minuten.

3. Kosmetische, nicht-therapeutische Verwendung von Harnstoff und/oder einem seiner Derivate in Mitteln zum Aufhellen keratinischer Fasern zur Penetrationsverbesserung von Wasserstoffperoxid in die keratinische Faser.

4. Kosmetische, nicht-therapeutische Verwendung von Harnstoff und/oder einem seiner Derivate in Mitteln zum Aufhellen keratinischer Fasern zur Reduktion der Schädigung in der keratinischen Faser durch Wasserstoffperoxid.

5. Mittel zur Aufhellung keratinischer Fasern, **dadurch gekennzeichnet, dass** es in einem kosmetischen Träger 0,01 bis 10 Gew.-%, bezogen auf das Gesamtgewicht des anwendungsbereiten Mittels, Harnstoff und eines seiner Derivate enthält.

6. Mittel nach Anspruch 5, **dadurch gekennzeichnet, dass** das Mittel zusätzlich 0,1 bis 3 Gew.-%, bevorzugt 0,3 bis 1,0 Gew.-%, jeweils bezogen auf das Gesamtgewicht des anwendungsbereiten Mittels, Ammoniumsulfat enthält.

7. Mittel nach einem der Ansprüche 5 oder 6, **dadurch gekennzeichnet, dass** das Mittel unmittelbar vor der Anwendung durch Vermischen aus einer Oxidationsmittelzubereitung B, enthaltend in einem kosmetischen Träger mindestens ein Oxidationsmittel, und einer Emulsionszubereitung A sowie gegebenenfalls einer Blondaktivatorzubereitung C hergestellt wird, wobei mindestens eine der Zubereitungen A, B oder C 0,01 bis 10 Gew.-%, bezogen auf das Gesamtgewicht des anwendungsbereiten Mittels, Harnstoff und eines seiner Derivate enthält.

8. Mittel nach Anspruch 7, **dadurch gekennzeichnet, dass** die Emulsionszubereitung A Harnstoff und/oder eines seiner Derivate zu 0,01 bis 10 Gew.-%, bezogen auf das Gesamtgewicht des anwendungsbereiten Mittels, enthält.

9. Mittel nach einem der Ansprüche 7 oder 8, **dadurch gekennzeichnet, dass** die Emulsionszubereitung A Ammoniumsulfat zu 0,3 bis 1,0 Gew.-%, bezogen auf das Gesamtgewicht des anwendungsbereiten Mittels, enthält.

10. Mittel nach einem der Ansprüche Anspruch 7 bis 9, **dadurch gekennzeichnet, dass** die Emulsionszubereitung A zusätzlich eine nichtionische Komponente mit einem HLB-Wert von mindestens 15 enthält.

11. Mittel nach einem der Ansprüche Anspruch 7 bis 10, **dadurch gekennzeichnet, dass** die Emulsionszubereitung A zusätzlich einen Pflegestoff, ausgewählt aus Vitaminen, Provitaminen und Vitaminvorstufen, insbesondere solche der Gruppen A, B₃, B₅, B₆, C, E, F und H, enthält.
